# EUROPÄISCHE PATENTSCHRIFT

(11) **EP 1 417 164 B1**
(45) Veröffentlichungstag und Bekanntmachung des Hinweises auf die Patenterteilung: **03.11.2010**
(21) Anmeldenummer: 02767312.8
(22) Anmeldetag: 02.08.2002
(51) Int. Cl.: C07C 51/25, C07C 51/215, C07C 51/235, C07C 51/43, C07C 51/487, C07C 51/44, C07C 57/05, C07C 57/07, C07C 57/075, C08F 220/00

(54) **VERFAHREN ZUR HERSTELLUNG WASSERABSORBIERENDER HARZE**
METHOD FOR PRODUCING WATER-ABSORBENT RESINS
PROCEDE POUR FABRIQUER DES RESINES HYDROPHILES

(30) Priorität: 03.08.2001 DE 10138150
(43) Veröffentlichungstag der Anmeldung: 12.05.2004
(73) Patentinhaber: BASF SE, 67056 Ludwigshafen (DE)
(72) Erfinder: FUCHS, Eberhard, 67227 Frankenthal (DE); MARTAN, Hans, 67227 Frankenthal (DE); NESTLER, Gerhard, A-1070 Wien (AT); MÜLLER-ENGEL, Klaus, Joachim, 76297 Stutensee (DE)
(86) Internationale Anmeldenummer: PCT/EP2002/008646
(87) Internationale Veröffentlichungsnummer: WO 2003/014172

(56) Entgegenhaltungen:
- EP-A- 0 770 592
- DE-A- 19 634 614
- FR-A- 2 753 445
- US-A- 3 868 417
- US-A- 5 426 221
- US-A- 5 482 597

## Beschreibung

Wasserabsorbierende Harze auf der Basis von Acrylsäure, insbesondere so genannte Superabsorber, finden in großem Umfang z.B. in Hygieneartikeln Verwendung.

Es ist bekannt, dass Acrylsäure durch heterogen katalysierte Gasphasenoxidation von Propan, Propylen (und/oder Acrolein) mit molekularem Sauerstoff an festen Katalysatoren hergestellt werden kann. Bei der Gasphasenoxidation von Propan, Propylen und/oder Acrolein entstehen neben der gewünschten Acrylsäure Nebenprodukte, insbesondere in Form von Carbonylverbindungen, wie z. B. Aldehyden, wie Benzaldehyd, Furfuralen, Propionaldehyd usw., sowie Ameisensäure und Maleinsäure bzw. Maleinsäureanhydrid, deren Beisein die Polymerisation beeinträchtigt, falls die Acrylsäure zur Herstellung von Superabsorbern verwendet wird. Aldehyde behindern die Polymerisation der Acrylsäure und führen außerdem zu verfärbten Polymeren. Die Gegenwart von Maleinsäureanhydrid führt bei der Polymerisation zur Bildung unerwünschter Copolymerisate, die die Eigenschaften der gewünschten Polymerisate beeinflussen. Verunreinigungen durch nicht polymerisationsfähige Carbonsäuren, wie Ameisensäure sind insbesondere dann nachteilig, wenn die durch Polymerisation hergestellten Polyacrylsäuren in Hygieneartikeln Verwendung finden, die mit der menschlichen Haut in Kontakt kommen, da diese Carbonsäuren äußerst hautreizend sind. Das Vorliegen oligomerer Acrylsäuren, z. B. Di-, Tri- oder Tetraacrylsäure, ist deshalb störend, weil sich bei der thermischen Behandlung der Polyacrylsäure die eingebaute oligomere Acrylsäure spaltet und Acrylsäure freisetzt, die ebenfalls äußerst hautreizend ist.

Bei der Gewinnung und/oder Isolierung von Acrylsäure werden in der Regel Prozess-Polymerisationsinhibitoren, wie Phenothiazin, Hydrochinon oder Hydrochinonmonomethylether, eingesetzt, um eine unerwünschte vorzeitige Polymerisation, insbesondere unter Einwirkung von Hitze, zu unterbinden. Unerwünschte Polymerisatbildung führt zu einer Belegung der Wärmetauscherflächen und Kolonnenböden sowie zu einem Verstopfen von Leitungen, Pumpen, Ventilen usw. Da die ausgeprägt inhibierend wirkenden Prozess-Polymerisationsinhibitoren naturgemäß auch die gezielte Herstellung von Polyacrylsäure beeinträchtigen, müssen sie aus der gewonnenen Acrylsäure anschließend wieder entfernt und durch in geringerem Umfang inhibierend wirkende Lagerpolymerisationsinhibitoren ersetzt werden.

Zur Entfernung bzw. Verminderung der genannten Nebenprodukte und Verunreinigungen werden im Stand der Technik in der Regel mehrstufige Destillations- und/oder Extraktions- und/oder Kristallisationsschritte angewendet. Dabei erhältliche zur Herstellung von Absorberharzen geeignete Acrylsäure wird in der Regel als Reinacrylsäure bezeichnet. So offenbart die EP 0 754 671 ein Destillationsverfahren zur Reinigung von mit Hilfe der katalytischen Oxidation von Propylen hergestellter Acrylsäure insbesondere zur Entfernung von Maleinsäureanhydrid. Die EP 0 727 408 offenbart eine zweistufige Destillation von Rohacrylsäure. Die Ameisensäure und Essigsäure enthaltende Kopffraktion wird zur Nutzbarmachung der Essigsäure verestert.

Die DE 2 241 714 offenbart ein Verfahren zur Abtrennung von Acrylsäure aus den Reaktionsgasen der Propylen- oder Acroleinoxidation unter Verwendung einer Gegenstromabsorption, wobei man aus der erhaltenen Absorptionslösung mit indifferenten Gasen Essigsäure und teilweise Wasser austreibt. Die DE 4 308 087 beschreibt ein Verfahren zur Abtrennung von Acrylsäure aus den Reaktionsgasen der katalytischen Propylen- und/oder Acroleinoxidation durch Gegenstromabsorption mit einer Mischung aus Diphenylether, Diphenyl und o-Dimethylphthalat.

Die DE 196 34 614 offenbart ein Verfahren zur destillativen Abtrennung von Rein(Meth)acrylsäure unter Verwendung einer Destillationsvorrichtung, die einen Dünnschichtverdampfer, einen Kondensator und eine eine Prallvorrichtung aufweisende Verbindung zwischen Dünnschichtverdampfer und Kondensator aufweist.

Der vorliegenden Erfindung liegt die Aufgabe zugrunde, ein Verfahren zur Herstellung wasserabsorbierender Harze anzugeben, wobei die Abtrennung von Acrylsäure aus den Reaktionsgasen der katalytischen Gasphasenoxidation von Propan, Propylen und/oder Acrolein technisch einfach erfolgt, und eine Acrylsäure liefert, die ohne weitere Reinigung unmittelbar zur Herstellung der wasserabsorbierenden Harze verwendet werden kann.

Erfindungsgemäß wird diese Aufgabe gelöst durch ein Verfahren zur Herstellung wasserabsorbierender Harze, bei dem man
a) Rohacrylsäure gewinnt, indem man entweder
   a1) Acrylsäure aus den Reaktionsgasen der katalytischen Gasphasenoxidation von Propan, Propylen und/oder Acrolein in einer Absorptionsflüssigkeit absorbiert und aus der mit Acrylsäure beladenen Absorptionsflüssigkeit Rohacrylsäure isoliert,
      oder
   a2) aus den Reaktionsgasen durch fraktionierte Kondensation eine Rohacrylsäurefraktion abtrennt, und die Fraktion gegebenenfalls einer kristallisativen Reinigung unterwirft,
b) die Rohacrylsäure mit einem Aldehydfänger behandelt,
c) aus der behandelten Rohacrylsäure destillativ Reinacrylsäure abtrennt, und
d) die Reinacrylsäure, gegebenenfalls nach Teilneutralisation, gegebenenfalls im Gemisch mit weiteren ethylenisch ungesättigten Monomeren, einer radikalischen Polymerisation unterwirft,
dadurch gekennzeichnet, dass man zur destillativen Abtrennung der Reinacrylsäure die behandelte Rohacrylsäure im Wesentlichen ohne Rücklauf von Kondensat thermisch in Acrylsäure-haltige Brüden und einen Rückstand trennt und die Brüden quantitativ zu Reinacrylsäure kondensiert, wobei man keine niedriger siedende Fraktion als die Reinacrylsäure-Fraktion isoliert.

Die in Schritt c) erhaltene Reinacrylsäure enthält in der Regel weniger als 1 ppm Prozess-Polymerisationsinhibitor, wie Phenothiazin, weniger als 5 ppm Ameisensäure, weniger als 5 ppm Aldehyde und weniger als 100 ppm Diacrylsäure.

Die Oxidation von Propan, Propylen und/oder Acrolein zur Acrylsäure in der Gasphase erfolgt auf an sich bekannte Weise. Der gegebenenfalls mit einem inerten Verdünnungsgas gemischte Zulauf wird im Gemisch mit Sauerstoff bei erhöhten Temperaturen, üblicherweise 200 bis 400 °C, sowie gegebenenfalls erhöhtem Druck über wenigstens einen heterogenen Katalysator, in der Regel übergangsmetallische, z. B. Molybdän, Vanadium, Wolfram und/oder Eisen enthaltende Mischoxidkatalysatoren geleitet und dabei oxidativ in Acrylsäure umgewandelt. Die Umsetzung kann einstufig oder zweistufig durchgeführt werden. Bei zweistufiger Reaktionsführung wird das Propylen in einer ersten Stufe zu Acrolein oxidiert und das Acrolein in einer zweiten Stufe zur Acrylsäure oxidiert. Als heterogene Katalysatoren sind in der ersten Stufe oxidische Mehrkomponenten-Katalysatoren auf der Basis der Oxide von Molybdän, Bismut und Eisen und in der zweiten Stufe entsprechende Katalysatoren auf der Basis der Oxide von Molybdän und Vanadium bevorzugt.

Die Umsetzung von Propan, Propylen und/oder Acrolein zu Acrylsäure ist stark exotherm. Der Zulaufstrom wird daher in vorteilhafter Weise mit einem inerten Verdünnungsgas, z. B. Luftstickstoff, Kohlendioxid, Methan und/oder Wasserdampf verdünnt. Obwohl die Art der verwendeten Reaktoren an sich keiner Beschränkung unterliegt, werden zweckmäßigerweise Rohrbündelwärmetauscher verwendet, die mit dem (oder den) Oxidationskatalysator(en) gefüllt sind, da bei diesen der überwiegende Teil der bei der Reaktion freiwerdenden Wärme durch Konvektion und Strahlung an die gekühlten Rohrwände abgeführt werden kann. Die bei der ein- oder zweistufigen katalytischen Gasphasenoxidation erhaltenen Reaktionsgase enthalten neben Acrylsäure üblicherweise nicht umgesetztes Propan, Propylen und/oder Acrolein, Wasserdampf, Kohlenmonoxid, Kohlendioxid, Stickstoff, Sauerstoff, Essigsäure, Propionsäure, Formaldehyd, weitere Aldehyde und Maleinsäure bzw. Maleinsäureanhydrid. Die Reaktionsgase enthalten typischerweise:
- Acrylsäure 1 bis 30 Gew.-%
- Propylen 0,05 bis 1 Gew.-%
- Acrolein 0,01 bis 2 Gew.-%
- Propan 0,01 bis 2 Gew.-%
- Wasserdampf 1 bis 30 Gew.-%
- Kohlenoxide 0,05 bis 15 Gew.-%
- Stickstoff 0 bis 90 Gew.-%
- Sauerstoff 0,05 bis 10 Gew.-%
- Ameisensäure 0,01 bis 1 Gew.-%
- Essigsäure 0,05 bis 2 Gew.-%
- Propionsäure 0,01 bis 2 Gew.-%
- Aldehyde 0,01 bis 3 Gew.-%
- Maleinsäureanhydrid 0,01 bis 0,5 Gew.-%

Aus den Reaktionsgasen wird zunächst sogenannte Rohacrylsäure mit einem Gehalt an Acrylsäure von üblicherweise wenigstens 99 Gew.-% isoliert. Verfahren zur Isolierung von Rohacrylsäure aus den Reaktionsgasen sind an sich bekannt. In einer Ausführungsform des erfindungsgemäßen Verfahrens absorbiert man Acrylsäure aus den Reaktionsgasen in einer Absorptionsflüssigkeit. Als Absorptionsflüssigkeit sind Flüssigkeiten geeignet, in denen Acrylsäure eine ausgeprägte Löslichkeit aufweist, z. B. höher als Acrylsäure siedende Flüssigkeiten (im Folgenden: "hochsiedende Flüssigkeit"), deren Siedepunkt vorzugsweise mehr als 160 °C (bei 1 atm) beträgt. Als hochsiedende Flüssigkeit kommen z. B. Diphenyl, Diphenylether, Dimethylphthalat, Ethylhexansäure, N-Methylpyrrolidon, Paraffinfraktionen oder Gemische davon in Frage. Alternativ können oligomere Acrylsäuren, wie Di-, Tri- und Tetraacrylsäure enthaltende Gemische, als hochsiedende Flüssigkeit eingesetzt werden. Diphenyl, Diphenylether, o-Dimethylphthalat oder Gemische davon sind bevorzugt, insbesondere ein Gemisch von 25 bis 30 Gew.-% Diphenyl und 70 bis 75 Gew.-% Diphenylether, das bezogen auf das Gemisch, 0,1 bis 25 Gew.-% o-Dimethylphthalat enthält.

Alternativ ist Wasser als Absorptionsflüssigkeit geeignet.

Die Absorptionsflüssigkeit wird mit den Reaktionsgasen in geeigneter Weise innig in Kontakt gebracht. Hierzu führt man die Reaktionsgase zweckmäßigerweise in einer Absorptionskolonne im Gegenstrom zu der absteigenden Absorptionsflüssigkeit. Als Absorptionskolonne kann man z. B. eine Füllkörper-, Packungs-, Ventilboden- oder Glockenbodenkolonne einsetzen.

Die Reaktionsgase, die in der Regel eine Temperatur von 200 bis 400 °C aufweisen, werden vor dem Einführen in die Absorptionskolonne vorzugsweise auf eine geeignete Absorptionstemperatur von beispielsweise 100 bis 180 °C abgekühlt. Das Abkühlen der Reaktionsgase auf die Absorptionstemperatur kann durch indirekte Kühlung, z. B. mittels eines Wärmetauschers, vorgenommen werden. Vorzugsweise erfolgt dieses Abkühlen jedoch durch direkten Kontakt mit einer Kühlflüssigkeit, vorzugsweise in einem Sprühwäscher. Die Kühlflüssigkeit wird vor Eintritt der Reaktionsgase in die Absorptionskolonne zweckmäßigerweise in einem Abscheider wieder weitgehend abgetrennt, gekühlt und rückgeführt. Die Kühlflüssigkeit ist vorzugsweise identisch mit der Flüssigkeit, die zur anschließenden Absorption der Acrylsäure aus den Reaktionsgasen verwendet wird.

Die mit Acrylsäure beladene Absorptionsflüssigkeit enthält neben Acrylsäure in der Regel noch flüchtige Verunreinigungen, wie Wasser, Acrolein, Formaldehyd sowie Ameisensäure und Essigsäure. Nebenkomponenten wie Wasser, Acrolein, Formaldehyd sowie die Essig- und Ameisensäure können insbesondere bei Einsatz einer hochsiedenden Flüssigkeit als Absorptionsflüssigkeit durch Strippen mit einem Strippgas zumindest teilweise entfernt werden. Hierzu wird die mit Acrylsäure beladene Absorptionsflüssigkeit in einer Desorptionskolonne im Gegenstrom zu einem Strippgas, wie z. B. Stickstoff oder Luft, geführt. Die erforderliche Strippgasmenge richtet sich vor allem nach der Desorptionstemperatur, die vorteilhaft 20 bis 50 °C höher gewählt wird, als die Absorptionstemperatur; man arbeitet vorzugsweise bei gleichem Druck wie im Absorptionsschritt. Die Strippgasmenge beträgt vorzugsweise, bezogen auf die Menge an Reaktionsgas, 5 bis 25 Vol.-%. Die Desorptionskolonne kann z. B. eine Füllkörper-, Packungs-, Ventilboden- oder Glockenbodenkolonne sein.

Die Kühlflüssigkeit und/oder die Absorptionsflüssigkeit enthalten üblicherweise z. B. in einer Menge von 0,01 bis 1 Gew.-% wenigstens eines Prozess-Polymerisationsinhibitors, wie Phenothiazin, phenolische Verbindungen wie Hydrochinon, Hydrochinonmonomethylether, p-Nitrosophenol, tert.-Butylphenole, 1-Oxyl-2,2,6,6-tetramethylpiperidin-4-ol oder Gemische davon. Häufig wird Phenothiazin in einer Menge von 0,01 bis 1 Gew.-% eingesetzt.

Aus der mit Acrylsäure beladenen Absorptionsflüssigkeit wird dann Rohacrylsäure isoliert. Bei Einsatz einer hochsiedenden Flüssigkeit als Absorptionsflüssigkeit erfolgt üblicherweise eine rektifikative Abtrennung der Rohacrylsäure. Die rektifikative Abtrennung erfolgt zweckmäßigerweise bei vermindertem Druck, z. B. 0,04 bis 0,1 bar, z. B. in einer Füllkörper- oder Bodenkolonne. Am Kopf oder im oberen Bereich der Rektifikationskolonne wird mit Vorteil ein Polymerisationsinhibitor zugesetzt. Die Rohacrylsäure kann dabei als Kopfprodukt abgenommen werden; vorzugsweise wird sie jedoch über einen Seitenabzug im oberen Bereich der Rektifikationskolonne abgenommen, wobei am Kopf der Kolonne geringe Mengen leichter als Acrylsäure siedender Verunreinigungen, wie Wasser und Essigsäure abgezogen werden. Die nach Abtrennung der Rohacrylsäure anfallende hochsiedende Flüssigkeit wird zweckmäßigerweise zurückgeführt und wieder zur Absorption verwendet. Unter Umständen ist es vorteilhaft, den hauptsächlich aus der hochsiedenden Flüssigkeit bestehenden Rückstand vor seiner Rückführung in die Absorptionskolonne ganz oder teilweise bei Temperaturen oberhalb 180 °C thermisch zu behandeln, wobei als Verunreinigung enthaltene esterartige oligomere Acrylsäuren aufspalten und die entstehende Acrylsäure gemeinsam mit der hochsiedenden Flüssigkeit abdestilliert. Die noch enthaltene Maleinsäure bzw. ihr Anhydrid können vor der Wiederverwendung der hochsiedenden Flüssigkeit in an sich üblicher Weise, z. B. durch Extraktion mit Wasser, entfernt werden.

Wird als Absorptionsflüssigkeit zur Absorption der Acrylsäure aus den Reaktionsgasen Wasser verwendet, so wird die Rohacrylsäure aus der primär erhaltenen wässrigen Acrylsäurelösung zweckmäßigerweise durch Extraktion mit einem Extraktionsmittel und anschließende Destillation des Extrakts isoliert. Das Extraktionsmittel soll einen hohen Verteilungskoeffizienten für Acrylsäure und eine geringe Löslichkeit in Wasser aufweisen und es muß ein Azeotrop mit Wasser bilden. Es können niedriger als Acrylsäure siedende Extraktionsmittel, wie Ethylacetat, Butylacetat, Ethylacrylat, 2-Butanon oder Gemische davon, oder höher als Acrylsäure siedende Extraktionsmittel, wie t-Butylphosphat, Isophoron oder aromatische Kohlenwasserstoffe, verwendet werden. Zur Extraktion führt man die wässrige Acrylsäurelösung geeigneterweise in einer Extraktionskolonne im Gegenstrom zu dem gewählten Extraktionsmittel.

Aus dem Extrakt wird dann destillativ Rohacrylsäure abgetrennt. Die Durchführung der Destillation richtet sich danach, ob ein höher oder niedriger als Acrylsäure siedendes Extraktionsmittel verwendet wird. Bei Einsatz eines niedriger als Acrylsäure siedenden Extraktionsmittels wird der Extrakt beispielsweise einer Lösungsmitteltrennkolonne zugeführt, in der das Extraktionsmittel und Restmengen Wasser über Kopf abdestilliert werden. Die Sumpffraktion der Lösungsmitteltrennkolonne wird dann einer Leichtsiederkolonne zugeführt, in der leichter als Acrylsäure siedende Verunreinigungen, wie Essigsäure über Kopf abgetrennt werden und als Sumpffraktion Rohacrylsäure anfällt.

Anstatt Rohacrylsäure durch Absorption in einer Absorptionsflüssigkeit aus den Reaktionsgasen zu isolieren, kann man Rohacrylsäure auch durch fraktionierte Kondensation der Reaktionsgase, gegebenenfalls mit anschließender kristallisativer Reinigung, gewinnen.

Zur fraktionierten Kondensation leitet man die Reaktionsgase, deren Temperatur vorzugsweise durch direkte Kühlung mit einer Kühlflüssigkeit auf z. B. 100 bis 180 °C verringert worden ist, zweckmäßigerweise in den unteren Bereich einer Kolonne mit trennwirksamen Einbauten ein und lässt sie innerhalb der Kolonne in sich aufsteigen. Über einen geeignet angebrachten Fangboden kann man als Mittelsiederfraktion eine Rohacrylsäurefraktion abnehmen. Ein derartiges Verfahren ist z. B. in der DE 19740253 oder der DE 19740252 beschrieben. In die Kolonne gibt man in der Regel einen Prozess-Polymerisationsinhibitor, wie die weiter oben erwähnten, zu.

Die bei der fraktionierten Kondensation erhaltene Rohacrylsäurefraktion kann zum Zwecke der Weiterreinigung einer Kristallisation zugeführt werden. Das Kristallisationsverfahren unterliegt keiner Beschränkung. Zweckmäßigerweise wird die kristallisative Reinigung, falls angewendet, als Suspensionskristallisation durchgeführt.

Die nach einer der vorstehenden Methoden erhaltene Rohacrylsäure wird mit einem Aldehydfänger behandelt. Die Zugabe des Aldehydfängers kann direkt in eine Rohrleitung, mittels welcher die Rohacrylsäure der weiteren Aufarbeitung zugeführt wird, oder in einen Verweilzeitbehälter erfolgen, in dem die Rohacrylsäure zwischengelagert wird, bevor sie der weiteren Aufarbeitung zugeführt wird.

Als Aldehydfänger sind alle Verbindungen geeignet, die die in der Rohacrylsäure enthaltenen Aldehyde im Wesentlichen quantitativ in Verbindungen mit einem höheren Siedepunkt als Acrylsäure umwandeln. Hierzu eignen sich besonders Stickstoffverbindungen mit wenigstens einer primären Aminogruppe. Als Beispiele lassen sich Aminoguanidinsalze, Hydrazin, Alkyl- und Arylhydrazine, Carbonsäurehydrazide oder Aminophenole aufführen. Davon ist Aminoguanidinhydrogencarbonat besonders bevorzugt. Der Aldehydfänger wird vorzugsweise im Überschuss zu dem in der Rohacrylsäure enthaltenen Aldehyd, z. B. in einer Menge von 1,5 bis 2,5 Mol pro Mol Aldehyd, eingesetzt. Zur Reaktion mit dem Aldehydfänger ist eine Temperatur von 15 bis 50 °C, vorzugsweise 20 bis 30 °C, geeignet. Üblicherweise hält man eine Reaktionsdauer von 10 Minuten bis 72 Stunden, vorzugsweise 2 bis 50 Stunden, ein. Wird Aminoguanidinhydrogencarbonat als Aldehydfänger eingesetzt, so bildet sich unter Kohlendioxidentwicklung zunächst Aminoguanidinhydrogenacrylat. Dieses reagiert mit den Aldehydgruppen der vorhandenen Aldehyde zu den entsprechenden Iminoguanidin-Derivaten bzw. deren Umlagerungsprodukten, die in der nachfolgenden Destillation als Schwersieder anfallen. Durch die Behandlung mit dem Aldehydfänger lässt sich der Restaldehydgehalt der Rohacrylsäure, ausgedrückt als Furfurol, auf unter 20 ppm, insbesondere unter 5 ppm, besonders bevorzugt unter 3 ppm, senken.

Aus der so behandelten Rohacrylsäure wird dann destillativ Reinacrylsäure abgetrennt. "Destillative Abtrennung" soll in diesem Zusammenhang weitestgehend verstanden werden und sowohl eine einfache Destillation, d.h. eine Destillation, bei der im wesentlichen kein Stoffaustausch zwischen Kondensat und Brüden erfolgt, als auch eine Rektifikation, bei der ein Teil des Kondensats im Gegenstrom zu den aufsteigenden Brüden geführt wird, umfassen. Es ist ein kritisches Merkmal der vorliegenden Erfindung, dass keine niedriger siedende Fraktion als die Reinacrylsäure-Fraktion isoliert wird, was zu einer Vereinfachung des Verfahrens und in der Regel zur Ersparnis einer Destillationskolonne ("Leichtsieder-Kolonne") führt. Zweckmäßigerweise geht man so vor, dass man die behandelte Rohacrylsäure thermisch in Acrylsäure-haltige Brüden und einen Rückstand trennt und die Brüden quantitativ zu Reinacrylsäure kondensiert.

Die thermische Trennung erfolgt vorzugsweise durch eine einfache Destillation, d. h. im Wesentlichen ohne Rücklauf von Kondensat. Man verwendet demzufolge zweckmäßigerweise eine Destillationskolonne ohne trennwirksame Einbauten, d. h. ein hohles säulen- oder turmförmiges Gebilde, das in der Regel aus Edelstahl gefertigt ist. Um zu verhindern, dass von den Acrylsäure-haltigen Brüden Tröpfchen von Rohacrylsäure mitgerissen werden, ist die Kolonne zweckmäßigerweise mit einem Tropfenabscheider üblicher Bauart, z. B. in Form einer Drahtgestrick-Packung mit großer innerer Oberfläche, die z.B. aus Chromnickelstählen, Aluminium, Polypropylen, Polytetrafluorethylen oder dergleichen gefertigt sein kann, oder in Form einer Füllkörperschüttung oder gerichteten Packung, z. B. eines Stapels beabstandet zueinander, parallel zur Längsachse der Kolonne angeordneter gewellter Bleche, geringer Höhe von z. B. 20 bis 100 cm ausgerüstet.

Die Sumpftemperatur liegt üblicherweise bei etwa 65 bis 130 °C, vorzugsweise 70 bis 100 °C, der Kolonnendruck bei 50 bis 120 mbar. Die Sumpfheizung der Kolonne erfolgt durch einen außen- oder innenliegenden Umlaufverdampfer, vorzugsweise einem Robert-Verdampfer oder einem Zwangsumlaufentspannungsverdampfer. Bei Verdampfern des Robert-Typs ist in einem zylindrischen Verdampferkörper ein Heizkörper mit senkrechten Siederohren untergebracht. Die Rohacrylsäure befindet sich im Innern der Siederohre. Die Zirkulation in den Rohren wird durch die aufsteigenden Dampfblasen bewirkt. Zur Rückführung der nach oben geförderten Flüssigkeit sind im Heizkörper ein oder mehrere Fallrohre angebracht.

Abgesehen von der beschriebenen Sumpfheizung wird die Kolonne vorzugsweise nicht aktiv beheizt; der Kolonnenmantel ist jedoch vorzugsweise isoliert, um einen übermäßigen Wärmeverlust durch Wärmestrahlung zu vermeiden. Der Verzicht auf eine Kolonnenheizung (abgesehen von der Sumpfheizung) bewirkt, dass Tröpfchen von Rohacrylsäure, die von den Acrylsäure-haltigen Brüden mitgerissen werden, auf ihrem Weg durch den Gasraum der Kolonne nicht erwärmt werden und sich ihre Größe durch Abdampfen flüchtiger Bestandteile nicht verringert. Die mitgerissenen Tröpfchen, die ihre Größe beibehalten oder durch Koagulation vergrößern, können dann bei der Passage der Acrylsäure-haltigen Brüden durch einen Tropfenabscheider gut zurückgehalten werden.

Abgesehen vom Kolonnenmantel sind die übrigen Teile der Anlage, die mit den Acrylsäure-haltigen Brüden in Kontakt kommen, insbesondere die Rohrleitungen, in denen die Arcylsäure-haltigen Brüden bis zu ihrer Kondensation geführt werden, mit einer Begleitheizung versehen, um eine unerwünschte vorzeitige Kondensation zu vermeiden. So können die Rohrleitungen z. B. als Doppelmantelrohr ausgeführt sein, in dessen Ringraum zwischen äußerem und inneren Mantel ein Heizmedium zirkuliert wird. Alternativ kann man ein von einem Heizmedium durchstromtes Rohr vorsehen, das mit der Rohrleitung, das die Acrylsäure-haltigen Brüden führt, in wärmeleitenden Kontakt steht, und z.B. spiralig um dieses gewickelt ist oder parallel dazu verläuft.

Eine bevorzugte Ausführungsform besteht darin, dass man die behandelte Rohacrylsäure in einer Kolonne mit Umlaufverdampfer in eine erste Menge Acrylsäure-haltiger Brüden und einen ersten Rückstand trennt, den ersten Rückstand in einem Filmverdampfer in eine zweite Menge Acrylsäure-haltiger Brüden und einen zweiten Rückstand trennt, die erste und zweite Menge Acrylsäure-haltiger Brüden vereinigt und zu Reinacrylsäure kondensiert, und den zweiten Rückstand verwirft.

Die Kolonne wird dabei vorzugsweise so betrieben, dass der erste Rückstand wenigstens 8 Gew.-%, z. B. 8 bis 30 Gew.-%, vorzugsweise 10 bis 25 Gew.-%, der der Destillationskolonne zugeführten Rohacrylsäure ausmacht. Auf diese Weise erreicht man, dass der Rückstand eine handhabbare, nicht zu hohe Viskosität aufweist. Außerdem wurde gefunden, dass es beim weitergehenden Eindampfen zu einer signifikant stärkeren Belagsbildung ("Fouling") auf den Wärmetauschflächen der zur Sumpfheizung der Kolonne herangezogenen Verdampfer kommt, die in kurzen Zeitabständen zum Abstellen und Reinigen der Anlage zwingt.

Zur Gewinnung der im ersten Rückstand noch enthaltenen Acrylsäure führt man diesen einem Filmverdampfer zu und erhält eine weitere Menge Acrylsäure-haltiger Brüden. Als Filmverdampfer sind Wischblattverdampfer besonders geeignet. Bei diesen Typen wird die einzudampfende Flüssigkeit durch eine rotierende Anordnung von Wischblättern über eine Röhrenwand verteilt. Verdampfer vom Sambay-Typ sind besonders bevorzugt. Es wurde gefunden, dass Filmverdampfer aufgrund ihrer Bauart eine geringere Neigung zur Belagsbildung zeigen und so ein stärkeres Eindampfen des Rückstands ohne Reinigungsunterbrechung erlauben, als dies in der primären Destillationskolonne möglich ist. Der erste Rückstand wird im Filmverdampfer vorzugsweise auf 35 Gew.-% bis 5 Gew.-%, insbesondere 10 Gew.-% bis 20 Gew.-%, aufkonzentriert.

Die zweite Menge Brüden wird mit der ersten Menge Brüden vereinigt, geeigneterweise, indem man die zweite Menge Brüden in die Destillationskolonne zurückführt. Zweckmäßigerweise führt man die zweite Menge Brüden unterhalb eines in der Destillationskolonne vorgesehenen Tropfenabscheiders ein und führt die vereinigten Brüden durch den Tropfenabscheider. Diese Verfahrensführung weist den Vorteil auf, dass zur Abtrennung mitgerissener Tröpfchen aus der ersten und der zweiten Menge Brüden nur ein gemeinsamer Tropfenabscheider erforderlich ist, was die Investitionskosten und den Reinigungsaufwand senkt. Die am Filmverdampfer anfallenden Rückstände, die z. B. 0,5 bis 5 Gew.-%, in der Regel 1 bis 2 Gew.-%, des gesamten Rohacrylsäurezulaufs entsprechen, werden verworfen.

In einer bevorzugten Ausführungsform des erfindungsgemäßen Verfahrens erwärmt man die mit dem Aldehydfänger behandelte Rohacrylsäure vor dem Einführen in die Destillationskolonne auf eine Temperatur von 40 bis 110 °C, vorzugsweise 50 bis 60 °C. Das Erwärmen erfolgt geeigneterweise durch indirekten Wärmetausch, z. B. mittels eines Durchlaufwärmetauschers. Das Einführen vorerwärmter Rohacrylsäure in die Destillationskolonne hat den Vorteil, dass im Kolonnensumpf eine geringere Wärmemenge über den dafür vorgesehenen Verdampfer aufgebracht werden muss, was wiederum zu einer verringerten Belagsbildung an dessen Wärmetauschflächen führt.

Bei der thermischen Trennung der Rohacrylsäure wird zweckmäßigerweise ein Spaltungskatalysator für esterartige oligomere Acrylsäure, insbesondere Diacrylsäure, mitverwendet, insbesondere Säuren wie Alkyl- und Arylsulfonsäuren, wie z. B. Dodecylbenzolsulfonsäure oder p-Toluolsulfonsäure, oder Basen, wie Natriumhydroxid oder Kaliumcarbonat. Der Spaltungskatalysator wird üblicherweise in einer Menge von 0,5 bis 10 kg pro Tonne Rohacrylsäure verwendet. Die Zugabe des Spaltungskatalysators kann zum Rohacrylsäurezulauf oder zum Zulauf des Filmverdampfers erfolgen.

Die Acrylsäure-haltigen Brüden werden dann zu Reinacrylsäure kondensiert, wobei die Kondensation durch indirekten Wärmeaustausch, z. B. in einem Wärmetauscher, oder vorzugsweise durch direkten Wärmetausch, z. B. in einem Gaskühler, durch direkten Kontakt mit einem Kühlmedium erfolgen kann. Als Kühlmedium wird vorzugsweise Reinacrylsäure verwendet. Die als Kühlmedium eingesetzte Reinacrylsäure enthält vorzugsweise einen Lager-Polymerisationsinhibitor, wie die oben genannten, z. B. in einer Menge von 10 bis 2000 ppm, vorzugsweise 25 bis 350 ppm. Hydrochinonmonomethylether ist zu diesem Zweck besonders bevorzugt. Die Kondensation der Acrylsäure-haltigen Brüden erfolgt üblicherweise bei einer Temperatur von 45 °C oder weniger, in der Regel bei 20 bis 40 °C.

Da die Bildung von Polymeren auch bei Verwendung eines Polymerisationsinhibitors nicht vollständig verhindert werden kann, kommt es vor allem bei längeren Laufzeiten zu Ablagerungen an Anlagenteilen, wie Reaktorwänden, Wärmetauscherflächen, auf Kolonnenböden und in Leitungen und Pumpen. Eine Reinigung der Anlage bzw. von Anlagenteilen und Apparaten ist daher von Zeit zu Zeit notwendig. Zu diesem Zweck behandelt man die Absorptionskolonne, die Destillationskolonne, den Filmverdampfer und/oder sonstige Anlagenteile, die mit den Reaktionsgasen, der mit Acrylsäure beladenen hochsiedenden Flüssigkeit, der Rohacrylsäure, den Acrylsäure-haltigen Brüden, den Rückständen oder der Reinacrylsäure in Kontakt kommen, periodisch mit einer wässrigen Baselösung.

Vorzugsweise wird der zu reinigende Anlagenteil entleert, gegebenenfalls mit Wasser vorgewaschen und mit der wässrigen Baselösung behandelt. Gemäß der Lehre der EP-A 1033359 spült man anschließend zweckmäßig mit Wasser. Es kann jedoch anstelle von Wasser auch eine wässrige Lösung eines Inhibitors verwendet werden. Bei dem zum Vor- und Nachspülen eingesetzten Wasser handelt es sich in der Regel um entsalztes Wasser, Kondensat oder Trinkwasser. Als Baselösung wird vorzugsweise eine 5 bis 25 gew.-%ige Kali- oder Natronlauge verwendet, wobei die Natronlauge bevorzugt ist. Die Reinigung mit der Baselösung erfolgt üblicherweise bei 20 bis 100 °C und dauert in der Regel 5 bis 20 Stunden. Die Art und die Menge der Ablagerungen bestimmen naturgemäß die Reinigungsbedingungen.

Besonders vorteilhaft ist eine abschließende Spülung mit einer 0,001 bis 1 gew.-%igen wässrigen Lösung eines Polymerisationsinhibitors. Als Polymerisationsinhibitoren werden vorzugsweise phenolische Verbindungen, z.B. Hydrochinon, Hydrochinonmonomethylether, tert.-Butylphenole oder Nitrosophenole, N-Oxylverbindungen wie 1-Oxyl-2,2,6,6-tetramethylpiperidin-4-ol, Methylenblau oder Gemische davon eingesetzt.

Falls eine Vorwäsche mit Wasser durchgeführt wird, wird die dabei anfallende Lösung vorteilhaft in den Aufarbeitungsprozess der Acrylsäure-haltigen Reaktionsgase der Gasphasenoxidation eingeschleust. Die Abwässer der Basebehandlung werden entsorgt oder aufgearbeitet und/oder ganz oder teilweise wiederverwendet. So kann die wässrige Lösung des Polymerisationsinhibitors vorteilhaft ganz oder teilweise mehrfach verwendet werden. Die Nachspülung mit der Inhibitorlösung verhindert weitgehend die Polymerbildung bei der Inbetriebnahme der Anlage und führt damit zu einer längeren Laufzeit der Anlage.

Die aufgeführte Art der Reinigung ist nicht auf die Anlagen zur Herstellung von Acrylsäure beschränkt, sondern kann auch bei Anlagen zur Herstellung/Reinigung von Methacrylsäure und von (Meth)acrylsäureestern vorteilhaft durchgeführt werden.

Besonders vorteilhaft ist es, die Kolonnen und sonstigen Apparateteile mit fest installierten Spülleitungen zu versehen, über die bei Bedarf die wässrige Baselösung, Wasser zur Vor- oder Nachwäsche bzw. die wässrige Inhibitorlösung zugeführt werden kann.

Die so erhaltene Reinacrylsäure ist unmittelbar zur Herstellung von Absorberharzen, insbesondere so genannter Superabsorber (Superabsorbent Polymers, kurz SAP) geeignet. Einen Überblick über die Herstellung von SAP auf Basis von Acrylsäure findet man in F.L. Buchholtz und A.T. Graham (Hrsg.) in "Modern Superabsorbent Technology", S. 69-117 und der dort zitierten Literatur.

Die Herstellung von SAP auf Basis von Acrylsäure erfolgt bekanntermaßen durch radikalische Polymerisation wässriger Monomerlösungen, die im Wesentlichen Acrylsäure und/oder deren Salze als polymerisierbare Monomere enthalten. Die Polymerisation erfolgt vorzugsweise als Lösungs- bzw. Gel-Polymerisation in homogener wässriger Phase oder als Suspensionspolymerisation, wobei die wässrige Monomerlösung die disperse Phase bildet. Die so erhaltenen Hydrogele werden anschließend oberflächennachvernetzt.

Zweckmäßigerweise führt man die Polymerisation als Lösungspolymerisation unter Ausnutzung des Trommsdorff-Norrish-Effektes (Gel-polymerisation) durch. Zu diesem Zweck wird eine wässrige, in der Regel 10 bis 70 gew.-%ige und vorzugsweise 20 bis 60 gew.-%ige wässrige Lösung einer Acrylsäure-haltigen Monomermischung, gegebenenfalls in Gegenwart einer geeigneten Pfropfgrundlage in Gegenwart einer Radikale bildenden Substanz polymerisiert.

In dem Polymerisationsverfahren wird die Acrylsäure-haltige Monomermischung in teil- oder vollständig neutralisierter Form eingesetzt, d. h. der Neutralisationsgrad aller Säuregruppen-tragenden Monomere liegt im Bereich von 20 bis 100 Mol-%.

Neben Acrylsäure kann die zu polymerisierende Monomermischung weitere ethylenisch ungesättigte Säuren, wie Methacrylsäure, Vinylsulfonsäure, 2-Acrylamido-2-methylpropansulfonsäure, oder neutrale ethylenisch ungesättigte Monomere, wie Acrylamid, Methacrylamid, N-Vinylamide, wie N-Vinylformamid, N-Vinylacetamid, N-Methylvinylacetamid, N-Vinylpyrrolidon und N-Vinylcaprolactam, enthalten. Die weiteren Monomere werden üblicherweise in einer Menge von weniger als 30 Gew.-%, bezogen auf Acrylsäure, mitverwendet.

In der Regel werden Vernetzermonomere mitverwendet, meist in einer Menge von 0,01 bis 5 Gew.-%, bezogen auf Acrylsäure. Als solche eignen sich N,N'-Methylenbisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantri(meth)acrylat, Ethylenglykoldi(meth)acrylat, Propylenglykoldi(meth)acrylat, Butandioldi(meth)acrylat, Hexandioldi(meth)acrylat, Triallylamin, Dialkyldiallylammoniumhalogenide, Divinylbenzol, Diallylphthalat, Polyethylenglykoldivinylether von Polyethylenglykolen eines Molekulargewichtes von 106 bis 4000, und dergleichen.

Geeignete Pfropfgrundlagen können natürlichen oder synthetischen Ursprungs sein. Hierzu zählen Stärken, d. h. native Stärken aus der Gruppe der Maisstärke, Kartoffelstärke, Weizenstärke, Reisstärke, Tapiokastärke, Sorghunstärke, Maniokstärke, Erbsenstärke oder deren Mischungen, modifizierte Stärken, Stärkeabbauprodukte, z. B. oxidativ, enzymatisch oder hydrolytisch abgebaute Stärken, Dextrine, z. B. Röstdextrine sowie niedere Oligo- und Polysaccharide, z. B. Cyclodextrine mit 4 bis 8 Ringgliedern. Als Oligo- und Polysaccharide kommen weiterhin Cellulose, Stärke- und Cellulosederivate in Betracht. Ferner eignen sich Polyvinylalkohole, Homo- und Copolymere des N-Vinylpyrrolidons, Polyamine, Polyamide, hydrophile Polyester oder Polyalkylenoxide, insbesondere Polyethylenoxid und Polypropylenoxid.

Als Polymerisationsreaktoren kommen die zur Herstellung üblichen Reaktoren, im Falle der Lösungspolymerisation insbesondere Bandreaktoren, Extruder und Kneter, in Betracht (siehe "Modern Superabsorbent Polymer Technology", Kapitel 3.2.3). Die Polymerisate werden besonders bevorzugt nach einem kontinuierlichen oder diskontinuierlichen Knetverfahren hergestellt.

Geeignete Initiatoren sind beispielsweise Peroxoverbindungen wie organische Peroxide, organische Hydroperoxide, Wasserstoffperoxid, Persulfate, Perborate, Azoverbindungen und die sogenannten Redoxkatalysatoren.

Die erhaltenen Polymerisate fallen in der Regel als Hydrogele an. Ihr Feuchtigkeitsgehalt liegt in der Regel im Bereich 20 bis 80 Gew.-%. Das so erhaltene Hydrogel wird dann in an sich bekannter Weise in ein Hydrogel bildendes Pulver überführt und anschließend oberflächennachvernetzt. Hierzu wird das bei der Polymerisation anfallende Hydrogel in der Regel zunächst nach bekannten Methoden zerkleinert. Die Grobzerkleinerung der Hydrogele erfolgt mittels üblicher Reiß- und/oder Schneidwerkzeuge. Das so erhaltene, vorzugsweise neutralisierte oder teilweise neutralisierte Polymerisat wird anschließend bei erhöhter Temperatur, z. B. im Bereich von 80 °C bis 250 °C. Hierbei erhält man die Polymerisate in Form von Pulvern oder Granulaten, die gegebenenfalls zur Einstellung der Partikelgröße noch mehreren Mahl- und Siebvorgängen unterworfen werden.

Die anschließende Oberflächennachvernetzung erfolgt in an sich bekannter Weise mit den so erhaltenen, getrockneten, vorzugsweise gemahlenen und abgesiebten Polymerpartikeln. Zur Oberflächenvernetzung werden Verbindungen eingesetzt, die wenigstens zwei funktionelle Gruppen aufweisen, die mit den funktionellen Gruppen, vorzugsweise den Carboxylgruppen des Polymers unter Vernetzung reagieren können (Nachvernetzungsmittel). Hierzu werden die Nachvernetzungsmittel, vorzugsweise in Form einer wässrigen Lösung auf die Oberfläche der Polymerisatpartikel aufgebracht.

Geeignete Nachvernetzungsmittel sind beispielsweise:
Ethylenglykoldiglycidylether, Bischlorhydrinether von Polyalkylenglykolen, Alkoxysilylverbindungen, Polyaziridine, Diole und Polyole sowie deren Ester mit Carbonsäuren oder mit Kohlensäure wie Ethylencarbonat oder Propylencarbonat, Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate, Di- und Poly-N-methylolverbindungen wie beispielsweise Methylenbis(N-methylol-methacrylamid) oder MelaminFormaldehyd-Harze.

Bei Bedarf können saure Katalysatoren wie p-Toluolsulfonsäure, Phosphorsäure, Borsäure oder Ammoniumdihydrogenphosphat zugesetzt werden.

Das Aufbringen der Vernetzer-Lösung erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt nachfolgen.

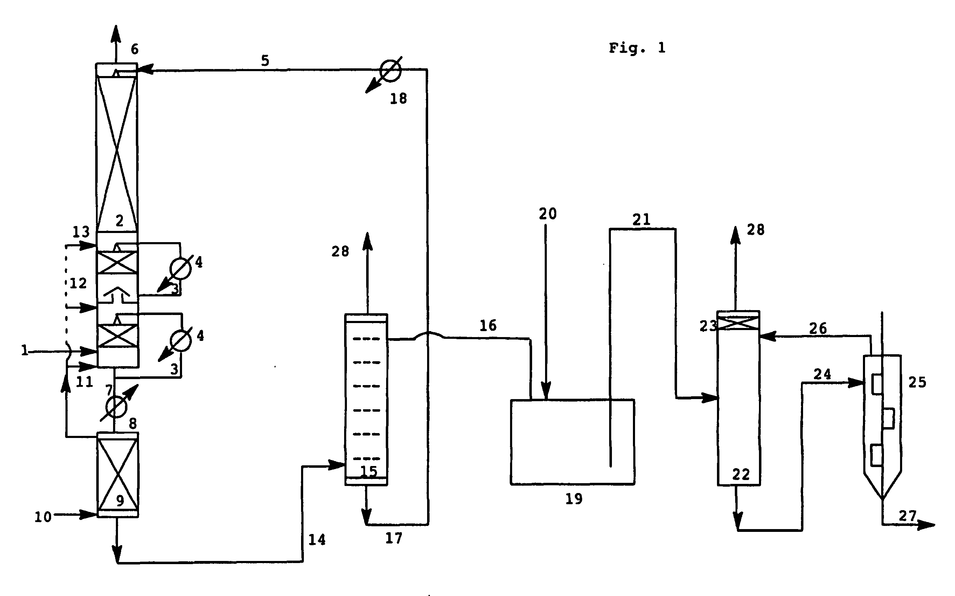

Eine bevorzugte Ausführungsform des erfindungsgemäßen Verfahrens wird anhand der beigefügten Figur 1 und den nachstehenden Beispielen näher erläutert.

Figur 1 zeigt eine zur Gewinnung von Reinacrylsäure geeignete Anlage. Das aus der Oxidationsstufe kommende heiße Reaktionsgas aus der Propan-, Propylen- und/oder Acroleinoxidation, das neben Acrylsäure Wasserdampf, Acrolein, Formaldehyd, Ameisensäure, Essigsäure, Maleinsäureanhydrid und Inertgase enthält, wird über Leitung 1 der Absorberkolonne 2 zugeleitet. In der Absorberkolonne 2 wird über die Flüssigkeitskreisläufe 3, die mit Wärmeaustauscher 4 ausgestattet sind, die für die Absorption gewünschte Temperatur eingestellt. Auf den Kopf der Absorberkolonne 2 wird über Leitung 5 die hochsiedende Flüssigkeit geleitet, die aus den Reaktionsgasen im Gegenstrom die Acrylsäure auswäscht. Die nicht absorbierten Bestandteile des Reaktionsgases verlassen die Absorberkolonne 2 über Leitung 6. Der Flüssigkeitsablauf der Absorberkolonne 2 wird über Leitung 7 unter Erwärmung im Wärmeaustauscher 8 auf den Kopf der Desorberkolonne 9 geleitet. Über Leitung 10 wird Strippgas in den Sumpf der Desorberkolonne 9 geführt. Mit dem Strippgas wird die mit Acrylsäure beladene hochsteigende Flüssigkeit im Gegenstrom behandelt, wobei flüchtige Verunreinigungen weitgehend entfernt werden. Das den Desorber am Kopf verlassende Gas wird über Leitung 11, gegebenenfalls über Leitung 12 oder 13, in die Absorberkolonne 2 geführt.

Der Ablauf der Desorberkolonne 9 wird über Leitung 14 der Destillationskolonne 15 zugeführt. Im oberen Bereich der Destillationskolonne 15 wird über Leitung 16 Rohacrylsäure abgezogen. Am Kopf der Destillationskolonne 15 werden über Leitung 28 restliche Leichtsieder entfernt. Die von Acrylsäure befreite hochsiedende Flüssigkeit kann, gegebenenfalls nach Aufarbeitung, nach Kühlung im Wärmetauscher 18 auf den Kopf der Absorberkolonne 2 zurückgeführt werden.

Die am Kopf der Destillationskolonne 15 abgezogene Rohacrylsäure wird über Leitung 16 dem Reaktionsgefäß 19 zugeleitet, in das über Leitung 20 ein Aldehydfänger dosiert wird. Nach angemessener Reaktionszeit wird die Rohacrylsäure über Leitung 21 in die Kolonne 22 geleitet, die von Einbauten frei ist und lediglich mit einem Tropfenabscheider 23 versehen ist. In der Leitung 21 ist in einer bevorzugten Ausführungsform ein Wärmetauscher zur Vorerwärmung der Rohacrylsäure vorgesehen. Die die Kolonne 22 verlassenden Acrylsäure-haltigen Brüden werden über Leitung 28 abgezogen und können zu Reinacrylsäure kondensiert werden. Der schwersiedende Rückstand am Sumpf der Kolonne 22 wird über Leitung 24 einem Eindampfer 25 zugeführt. Die anfallenden Brüden werden über Leitung 26 zurück in die Kolonne 22 geführt und unterhalb des Tropfenabscheiders 23 eingeleitet. Der eingedampfte Rückstand aus dem Eindampfer 25 wird über Leitung 27 abgezogen und entsorgt.

### Beispiel 1

Gemäß Fig. 1 wurden die heißen Reaktionsgase (230 °C) der Propenoxidation, die auf an sich bekannte Weise mit Luftsauerstoff zweistufig in der Gasphase an Multimetalloxidkatalysatoren durchgeführt wurde, über Leitung 1 der Absorberkolonne 2 zugeführt. Die Kolonne war mit 35 Glockenböden und zwei außenliegenden Wärmetauschern ausgerüstet. Am Kopf der Kolonne wurden 0,7 kg eines Lösemittelgemisches, bestehend aus 58,8 Gew.-% Diphenylether, 21,2 Gew.-% Diphenyl und 20 Gew.-% o-Dimethylphthalat, das 0,1 Gew.-% Phenothiazin enthielt pro 1000 1 Reaktionsgase mit einer Temperatur von 45 °C zugeführt (Leitung 5). Die Wärmetauscher wurden in einer Weise betrieben, dass die Gastemperatur nach dem 2. Wärmetauscher etwa 60 °C betrug. Der Acrylsäure-haltige Sumpfablauf der Kolonne wurde mit Hilfe des Wärmetauschers 8 auf 105 °C eingestellt und der Desorberkolonne 9 (25 Glockenböden) zugeführt. Von unten wurden 200 l auf 90 °C erwärmte Luft pro kg Sumpfablauf eingeblasen. Die mit Leichtsiedern beladenen Strippgase wurden wieder der Absorptionskolonne zugeführt. Der Ablauf der Kolonne 9 wurde der Destillationskolonne 15 (43 Dual-Flow-Böden, außenliegender Umlaufverdampfer, Sumpftemperatur 175 °C, Kopfdruck 100 mbar) auf dem 5. Boden zugeführt. Über einen Seitenabzug (35 Böden) wurde flüssig eine Roh-Acrylsäure ausgeschleust, die unter anderen folgende Komponenten enthielt:

| | |
|---|---|
| Acrylsäure | 99,3 Gew.-% |
| Diacrylsäure | 0,2 Gew.-% |
| Essigsäure | 0,15 Gew.-% |
| Propionsäure | 0,04 Gew.-% |
| Furfurale | 0,04 Gew.-% |
| Benzaldehyd | 0,01 Gew.-% |
| Wasser | 0,1 Gew.-% |
| Phenothiazin | 0,05 Gew.-% |

Die am Kopf der Kolonne ausgeschleusten Leichtsieder, hauptsächlich Wasser und Essigsäure, enthielten 2 Gew.-% Acrylsäure, das im Sumpf der Kolonne anfallende Lösungsmittelgemisch enthielt etwa 1 Gew.-% Acrylsäure und wurde in die Absorptionskolonne zurückgeführt. Die Rohacrylsäure wurde in einem Rührbehälter (19) mit der doppelten molaren Menge (bezogen auf Furfural und Benzaldehyd) an Aminoguanidinhydrogencarbonat bei 23 °C 10 Stunden behandelt. Anschließend wurde das Gemisch auf 50 °C erwärmt, mit 0,3 Gew.-% Dodecylbenzolsulfonsäure versetzt und dem Sumpf der Destillationskolonne 22 zugeführt. Die Kolonne war mit einem Spritzschutz (23) und einem Umlaufverdampfer ausgerüstet. Die Sumpftemperatur betrug 85 °C, der Druck 90 mbar. Die gasförmig ausgeschleuste Acrylsäure wurde durch Quenchen mit flüssiger Acrylsäure kondensiert und durch Zusatz von 200 ppm Hydrochinonmonomethylether (MEHQ) stabilisiert. Hierzu wurde eine etwa 1,5 gew.-%ige Lösung von MEHQ in Acrylsäure kontinuierlich in der erforderlichen Menge zudosiert.

Aus dem Sumpfprodukt (15 Gew.-% des Zulaufs) wurde in einem Sambay-Verdampfer (25) die restliche Acrylsäure weitgehend abdestilliert (75 °C, 70 mbar), wobei die Brüden unterhalb des Spritzschutzes (23) in die Kolonne (22) eingeleitet wurden.

Die kondensierte Reinacrylsäure hatte folgende Zusammensetzung:

| | |
|---|---|
| Acrylsäure | 99,7 Gew.-% |
| Diacrylsäure | 0,01 Gew.-% |
| Essigsäure | 0,14 Gew.-% |
| Propionsäure | 0,04 Gew.-% |
| Aldehyde | < 5 ppm |
| Phenothiazin | < 1 ppm |
| Wasser | 0,11 Gew.-% |

Der Sumpf der Sambay-Destillation (etwa 2 Gew.-% des Zulaufs zur Kolonne 22) enthielt ca. 20 Gew.-% Acrylsäure und wurde entsorgt. Es wurden etwa 99 Gew.-% der in der Rohacrylsäure enthaltenen Acrylsäure isoliert. Die Destillationseinheit konnte mehr als 30 Tage problemlos betrieben werden.

Aus der Reinacrylsäure wurde eine 40 gew.-%ige wässrige Lösung hergestellt, die mit Natronlauge neutralisiert wurde. Die Lösung wurde mit 0,50 Gew.-%, bezogen auf Acrylsäure, Vernetzer (Diacrylat eines Polyethylenglycols mit einem mittleren Molekulargewicht von 400) versetzt. Zur Initiierung wurde folgendes System verwendet:

| | | |
|---|---|---|
| 0,005 | Gew.-% | Wasserstoffperoxid und |
| 0,006 | Gew.-% | Ascorbinsäure und |
| 0,28 | Gew.-% | Natriumperoxodisulfat, |

bezogen auf Acrylsäure.

Die einzelnen Komponenten dieser Reaktionslösung (verdünnte wässrige Lösungen von Wasserstoffperoxid, Ascorbinsäure, Natriumperoxodisulfat und die Monomer/Vernetzerlösung) wurden getrennt in einen Knetreaktor eindosiert und dort während des Einlaufens im Reaktor gemischt, wobei die Polymerisation schon während des Mischens zügig startete.

Es wurden 600 kg/h Reaktionslösung eingebracht, und das im Kneter durch Polymerisation erzeugte Gel wurde kontinuierlich ausgetragen. Die Temperatur des Kühlwassers im Reaktormantel wurde auf 90 °C geregelt. Während der Polymerisation wurden 14 m³/h Stickstoff als Inertgas durch den Kneter geführt. Das Reaktionsvolumen betrug 300 1.

Das ausgetragene Gel wurde getrocknet, gemahlen und durch Sieben eine Korngrößenfraktion von 100 - 800 µm erhalten.

## Patentansprüche

1. Verfahren zur Herstellung wasserabsorbierender Harze, bei dem man
a) Rohacrylsäure gewinnt, indem man entweder
a1) Acrylsäure aus den Reaktionsgasen der katalytischen Gasphasenoxidation von Propan, Propylen und/oder Acrolein in einer Absorptionsflüssigkeit absorbiert und aus der mit Acrylsäure beladenen Absorptionsflüssigkeit Rohacrylsäure isoliert,
oder
a2) aus den Reaktionsgasen durch fraktionierte Kondensation eine Rohacrylsäurefraktion abtrennt, und die Fraktion gegebenenfalls einer kristallisativen Reinigung unterwirft,
b) die Rohacrylsäure mit einem Aldehydfänger behandelt,
c) aus der behandelten Rohacrylsäure destillativ Reinacrylsäure abtrennt, und
d) die Reinacrylsäure, gegebenenfalls nach Teilneutralisation, gegebenenfalls im Gemisch mit weiteren ethylenisch ungesättigten Monomeren, einer radikalischen Polymerisation unterwirft,
**dadurch gekennzeichnet, dass** man zur destillativen Abtrennung der Reinacrylsäure die behandelte Rohacrylsäure im Wesentlichen ohne Rücklauf von Kondensat thermisch in Acrylsäure-haltige Brüden und einen Rückstand trennt und die Brüden quantitativ zu Reinacrylsäure kondensiert, wobei man keine niedriger siedende Fraktion als die Reinacrylsäure-Fraktion isoliert.

2. Verfahren nach Anspruch 1, wobei man die mit Acrylsäure beladene Absorptionsflüssigkeit zur Entfernung flüchtiger Verunreinigungen mit einem Strippgas strippt.

3. Verfahren nach Anspruch 1 oder 2, wobei man als Aldehydfänger Aminoguanidinhydrogencarbonat verwendet.

4. Verfahren nach einem der vorhergehenden Ansprüche, wobei man die behandelte Rohacrylsäure in einer Kolonne mit Umlaufverdampfer in eine erste Menge Acrylsäure-haltiger Brüden und einen ersten Rückstand trennt, den ersten Rückstand in einem Filmverdampfer in eine zweite Menge Acrylsäure-haltiger Brüden und einen zweiten Rückstand trennt, die erste und zweite Menge Acrylsäure-haltiger Brüden vereinigt und zu Reinacrylsäure kondensiert, und den zweiten Rückstand verwirft.

5. Verfahren nach einem der vorhergehenden Ansprüche, wobei die Absorptionsflüssigkeit einen Polymerisationsinhibitor enthält.

6. Verfahren nach einem der vorhergehenden Ansprüche, wobei man als Absorptionsflüssigkeit Diphenylether, Diphenyl, ortho-Dimethylphthalat oder Gemische davon verwendet.

## Claims

1. A process for preparing water-absorbent resins, which comprises
a) obtaining crude acrylic acid by either
a1) absorbing acrylic acid from the reaction gases from the catalytic gas-phase oxidation of propane, propylene and/or acrolein in an absorption liquid and isolating crude acrylic acid from the absorption liquid laden with acrylic acid,
or
a2) separating a crude acrylic acid fraction from the reaction gases by fractional condensation and, if desired, subjecting the fraction to purification by crystallization,
b) treating the crude acrylic acid with an aldehyde scavenger,
c) separating pure acrylic acid from the treated crude acrylic acid by distillation, and
d) subjecting the pure acrylic acid, if desired after partial neutralization, if desired in admixture with further ethylenically unsaturated monomers, to a free-radical polymerization,
wherein the isolation of the pure acrylic acid by distillation is carried out substantially without reflux of condensate by thermally separating the treated crude acrylic acid into vapor comprising acrylic acid and a residue and quantitatively condensing the vapor to obtain pure acrylic acid, no fraction boiling lower than the pure acrylic acid fraction being isolated.

2. The process according to claim 1, wherein the absorption liquid laden with acrylic acid is stripped by means of a stripping gas to remove volatile impurities.

3. The process according to claim 1 or 2, wherein the aldehyde scavenger used is aminoguanidine hydrogen carbonate.

4. The process according to any of the preceding claims, wherein the treated crude acrylic acid is separated in a column provided with a circulation vaporizer into a first quantity of vapor comprising acrylic acid and a first residue, the first residue is separated in a film evaporator into a second quantity of vapor comprising acrylic acid and a second residue, the first and second quantities of vapor comprising acrylic acid are combined and condensed to give pure acrylic acid, and the second residue is discarded.

5. The process according to any of the preceding claims, wherein the absorption liquid comprises a polymerization inhibitor.

6. The process according to any of the preceding claims, wherein the absorption liquid used is diphenyl ether, biphenyl, dimethyl ortho-phthalate or a mixture thereof.

## Revendications

1. Procédé de fabrication de résines absorbant l'eau, selon lequel
a) de l'acide acrylique brut est obtenu
a1) en absorbant de l'acide acrylique à partir des gaz de réaction de l'oxydation catalytique en phase gazeuse de propane, propylène et/ou acroléine dans un liquide d'absorption et en isolant de l'acide acrylique brut à partir du liquide d'absorption chargé en acide acrylique
ou
a2) en séparant une fraction acide acrylique brut des gaz de réaction par condensation fractionnée et en soumettant éventuellement la fraction à une purification par cristallisation,
b) l'acide acrylique brut est traité avec un capteur d'aldéhyde,
c) de l'acide acrylique pur est séparé par distillation de l'acide acrylique brut traité, et
d) l'acide acrylique pur, éventuellement après une neutralisation partielle, éventuellement en mélange avec d'autres monomères éthyléniquement insaturés, est soumis à une polymérisation radicalaire,
**caractérisé en ce que** pour la séparation par distillation de l'acide acrylique pur, l'acide acrylique brut traité est séparé thermiquement essentiellement sans reflux de condensat en vapeurs contenant de l'acide acrylique et en un résidu, et les vapeurs sont condensées quantitativement en acide acrylique pur, aucune fraction de point d'ébullition inférieur à la fraction acide acrylique pur n'étant isolée.

2. Procédé selon la revendication 1, dans lequel le liquide d'absorption chargé en acide acrylique est extrait avec un gaz d'extraction pour éliminer les impuretés volatiles.

3. Procédé selon la revendication 1 ou 2, dans lequel de l'hydrogénocarbonate d'aminoguanidine est utilisé en tant que capteur d'aldéhyde.

4. Procédé selon l'une quelconque des revendications précédentes, dans lequel l'acide acrylique brut traité est séparé dans une colonne munie d'un évaporateur à circulation en une première quantité de vapeurs contenant de l'acide acrylique et en un premier résidu, le premier résidu est séparé dans un évaporateur à film en une seconde quantité de vapeurs contenant de l'acide acrylique et un second résidu, la première et la seconde quantité de vapeurs contenant de l'acide acrylique sont réunies et condensées en acide acrylique pur, et le second résidu est évacué.

5. Procédé selon l'une quelconque des revendications précédentes, dans lequel le liquide d'absorption contient un inhibiteur de polymérisation.

6. Procédé selon l'une quelconque des revendications précédentes, dans lequel le diphényléther, le diphényle, l'orthophtalate de diméthyle ou leurs mélanges sont utilisés en tant que liquide d'absorption.
